# EUROPÄISCHE PATENTSCHRIFT

(11) **EP 2 762 126 B1**
(45) Veröffentlichungstag und Bekanntmachung des Hinweises auf die Patenterteilung: **04.04.2018**
(21) Anmeldenummer: 14153390.1
(22) Anmeldetag: 31.01.2014
(51) Int. Cl.: A61K 8/35, A61Q 3/02, A61K 8/55, A61K 8/60, A61K 8/81, A61K 8/87, A61K 8/73, A61K 8/04

(54) **Leicht ablösbare Nagellack-Zusammensetzung**
Easily removed nail varnish composition
Composition de vernis à ongles à dissolution facile

(30) Priorität: 01.02.2013 DE 102013101035
(43) Veröffentlichungstag der Anmeldung: 06.08.2014
(73) Patentinhaber: CosTrade Beauty Consulting GmbH, 6840 Götzis (AT)
(72) Erfinder: Koschar, Mirko, 6844 Altach (AT)
(74) Vertreter: Rössler, Matthias

(56) Entgegenhaltungen:
- DE-A1-102011 102 661
- US-A- 3 477 969
- US-A- 5 516 509
- US-A1- 2012 199 151

## Beschreibung

Die vorliegende Erfindung betrifft eine Zusammensetzung, ein System zur Behandlung eines Finger- oder Zehennagels sowie ein Verfahren zur kosmetischen Behandlung eines Finger- oder Zehennagels.

Nagellack ist weltweit besonders bei Frauen beliebt und entsprechend verbreitet. Er wird in streichfähigem Zustand auf die von der Oberhaut an den streckseitigen Enden der Finger und Zehen gebildeten Hornplatten, den so genannten Nagelplatten, aufgetragen und dient nach seiner Aushärtung vornehmlich kosmetischen Zwecken, kann aber eine Schutzfunktion des Nagels übernehmen. Zur Dekoration verleiht der Nagellack dem Nagel ein besonderes Aussehen, z. B. eine besondere Farbe und/oder einen besonderen Glanz.

Herkömmliche Nagellacke bestehen aus Lösungen, Suspensionen oder Emulsionen bestimmter Substanzen wie z. B. Nitrocellulose oder verschiedenen synthetischen Polymeren (z. B. Polyacrylate, Alkydharze oder Polyvinylacetat) in geeigneten Lösungsmitteln (z. B. Alkohole, Ester, Ketone), die mit Additiven zur Gestaltung des optischen Erscheinungsbildes, z. B. löslichen Farbstoffen, unlöslichen Farbpigmenten oder Effektstoffen (z. B. zur Erzeugung von Perlmutt- oder Metalleffekten) versetzt sind. Zusätzlich können in der Mischung Weichmacher (wie z. B. Kampfer oder Dibutylphtalat) und Verschnittstoffe (z. B. Toluol oder Xylol) enthalten sein. Derartige Nagellacke härten durch das Verdunsten des Lösungsmittels sowie der anderen flüchtigen Bestandteile aus.

Der Nachteil derartiger Nagellacke besteht jedoch unter anderem darin, dass nach dem Auftragen des Lackes eine Trocknungszeit von mehreren Minuten bis zu einer halben Stunde einzuhalten ist. In dieser Zeit ist der Lack sehr empfindlich gegen Berührung, sodass zur Erzielung eines ansprechenden Erscheinungsbildes die Finger bzw. Zehen in einer Schutzhaltung gehalten werden müssen. Bei mehrmaligem Auftragen des Lackes in Schichten ist diese Zeit sogar deutlich verlängert.

Zur Lösung dieses Problems schlägt beispielsweise die DE-A-100 23 298 ein Nagellacksystem vor, dessen Aushärtungsprozess nicht auf Verdunstung beruht, sondern durch die Bestrahlung mit elektromagnetischen Wellen bzw. Quanten - vorzugsweise in Gestalt von Licht - zu einem beliebigen Zeitpunkt nach dem Auftragen initiierbar ist. Hierzu wird insbesondere die Applikation ultravioletter Strahlung vorgeschlagen. Ein solches Nagellacksystem ist beispielsweise in der DE-A-10 2005 063 061 A1 beschrieben und umfasst ein polymerisierbares Mehrkomponentensystem, welches ethylenisch ungesättigte Monomere, wie etwa (Meth)Acrylsäure oder (Meth)Acrylsäureester, einen mindestens zwei ethylenisch ungesättigte Gruppen aufweisenden Vernetzer, wie beispielsweise Triethylenglykoldimethacrylat und/oder Urethan-Oligomer-(Meth)Acrylate oder Polyester-Urethan-Oligmer-(Meth)acrylate, sowie Photoinitiatoren beinhaltet.

Gegenüber Nagellacksystemen, die durch das Verdunsten von Lösungsmitteln aushärten, zeichnen sich die in der DE-A-100 23 298 oder der DE-A-10 2005 063 061 A1 beschriebenen Nagellacksysteme durch eine deutlich schnellere Aushärtung aus. Nachteilig an solchen durch UV-Licht aushärtbaren Nagellacksystemen ist allerdings, dass sie sich teilweise nur schwer wieder von der Nageloberfläche entfernen lassen. Ein solches Entfernen ist jedoch in der Regel erwünscht, um beispielsweise die Finger- oder Zehennägel in anderer Weise optisch zu gestalten.

Eine verbesserte Entfernung der ausgehärteten Nagellacksysteme wurde in der DE-A-102011102661 vorgeschlagen. Diese Schrift offenbart Nagellacksysteme, die dreischichtige Lackierungen ergeben. Die Beschichtung umfasst eine Grund-, eine Farb- sowie eine Glanzschicht. Die Lacksysteme umfassen unter anderem ein Disaccharidester zur Verbesserung von Glanz und Haftung. Die Nagellackierungen sind mittels Lösungsmitteln zu entfernen. Es wäre jedoch wünschenswert, das Entfernen des gehärteten Lacks ohne den Einsatz von Lösungsmitteln vornehmen zu können.

Der vorliegenden Erfindung lag die Aufgabe zugrunde, die sich aus dem Stand der Technik ergebenden Nachteile im Zusammenhang mit Nagellacken zu überwinden.

Insbesondere lag der vorliegenden Erfindung die Aufgabe zugrunde, ein Nagellacksystem anzugeben, dessen Aushärtung auf der Finger- oder Zehennageloberfläche kontrolliert initiiert werden kann, welches besonders schnell unter Ausbildung einer besonders kratzfesten und gut haftenden Beschichtung ausgehärtet werden kann und welches trotz dieser guten Haftung besonders leicht wieder ohne die Behandlung mit Lösungsmitteln entfernt werden kann. Der ausgehärtete Lackfilm sollte sich wie eine Art Klebefolie abziehbar und rückstandslos entfernbar sein.

Auch lag der vorliegenden Erfindung die Aufgabe zugrunde, ein Verfahren zur kosmetischen Behandlung eines Finger- oder Zehennagels anzugeben, mit dem farbige und glänzende Nagellackschichten in kontrollierter und schneller Weise unter Erhalt von kratzfesten und gut haftenden Schichten aufgebracht werden können, die ohne Beeinträchtigung des Erscheinungsbildes für längere Zeit, vorzugsweise für 15 bis 20 Tage getragen werden können und die sich anschließend besonders leicht ohne Lösungsmittel wie eine Art Klebefolie entfernen lassen.

Einen Beitrag zur Lösung der eingangs genannten Aufgaben leistet eine Zusammensetzung, beinhaltend die Komponenten
a) 25 bis 95 Gew.-% eines durch elektromagnetische Strahlung härtbaren Mehrkomponentensystems, umfassend
   a1) mindestens ein eine ethylenisch ungesättigte Gruppe aufweisendes Monomer,
   a2) mindestens ein aliphatisches, difunktionelles PolyesterUrethan-Oligomer-(Meth)Acrylat als mindestens zwei ethylenisch ungesättigte Gruppen aufweisender Vernetzer, sowie
   a3) mindestens einen Photoinitiator,
b) 0 bis 4 Gew.-% eines Disaccharidesters,
c) 0 bis 30 Gew.-% Lösungsmittel, und
d) 0 bis 50 Gew.-% von den Komponenten a) bis c) verschiedene Additive,
wobei die Mengenangaben jeweils auf das Gesamtgewicht der Zusammensetzung bezogen sind und wobei die Summe der Komponenten a) bis d) 100 Gew.-% beträgt, und wobei die Zusammensetzung 0,3 bis 5 Gew.-% Nitrozellulose als filmbildendes Additiv gemäß Komponente d) beinhaltet.

Die erfindungsgemäße Zusammensetzung beinhaltet als Komponente a) ein durch elektromagnetische Strahlung härtbares Mehrkomponentensystem, beinhaltend mindestens ein eine ethylenisch ungesättigte Gruppe aufweisendes Monomer a1), mindestens ein aliphatisches, difunktionelles Polyester-Urethan-Oligomer-(Meth)Acrylat als mindestens zwei ethylenisch ungesättigte Gruppen aufweisender Vernetzer a2) sowie mindestens einen Photoinitiator a3). Als durch elektromagnetische Strahlung härtbares Mehrkomponentensystem a) ist dabei ein durch UV-Licht härtbares Mehrkomponentensystem besonders bevorzugt.

Bei dem mindestens einen eine ethylenisch ungesättigte Gruppe aufweisenden Monomer a1) handelt es sich vorzugsweise um ein ethylenisch ungesättigtes Monomer, welches eine Carbonsäure-Gruppe oder eine Carbonsäureester-Gruppe aufweist. Erfindungsgemäß bevorzugte Monomere a1) basieren auf einem (Meth)Acrylat und sind ausgewählt aus der Gruppe beinhaltend (Meth)Acrylsäure, (Meth)Acrylsäureester wie beispielsweise (Meth)Acrylsäure-methylester oder (Meth)Acrylsäureethylester oder, erfindungsgemäß besonders bevorzugt, um Ester aus Diolen und (Meth)Acrylsäure wie beispielsweise Hydroxymethyl(meth)acrylat, Hydroxyethyl(meth)acrylat oder Hydroxypropyl(meth)-acrylat, wobei der Einsatz von Hydroxypropylmethacrylat und Hydroxyethylacrylat, insbesondere Hydroxyethylmethacrylat als Komponente a1) erfindungsgemäß am meisten bevorzugt ist. "(Meth)Acrylsäure" wird in diesem Text für die Verbindungen mit den Nomenklaturnamen "Methacrylsäure" und "Acrylsäure" verwendet.

Die Zusammensetzung kann mindestens einen weiteren Vernetzer a2) enthalten. Bei dem mindestens einen weiteren mindestens zwei ethylenisch ungesättigte Gruppen aufweisenden Vernetzer a2) handelt es sich vorzugsweise um Alkenyldi(meth)acrylate, beispielsweise Ethylenglykoldi(meth)acrylat, 1,3-Propylengly-koldi(meth)acrylat, 1,4-Butylenglykoldi(meth)acrylat, 1,3-Butylenglykoldi(meth)-acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxyethylen)bis(meth)acrylamid, N,N'-Hexamethylenbis-(meth)acrylamid oder N,N'-Methylenbis(meth)acrylamid, Polyalkoxydi(meth)acrylate, beispielsweise Diethylenglykoldi(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi(meth)acrylat, Bisphenol-A-di(meth)acrylat, ethoxyliertes Bisphenol-A-di(meth)acrylat, Benzylidindi(meth)-acrylat, 1,3-Di(meth)acryl-oyloxypropanal-2, Hydrochinondi(meth)acrylat, Di-(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)acrylat, Thiopropylenglykoldi(meth)acrylat, Thiopolyethylenglykoldi(meth)acrylat, Thiopolypropylenglykoldi(meth)acrylat, Bis(2-Meth-acryloxyethyl)-N,N'-nonylen-biscarbamat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)-allylverbindungen, beispielsweise Di(meth)allylphthalat oder Di(meth)allylsuccinat, Homo- und Copolymere von Di(meth)allyl-dimethylammoniumchlorid und Homo- und Copolymere von Diethyl(meth)-allylamino-methyl(meth)-acrylat-ammoniumchlorid, Vinyl-(meth)acryl-Verbindun-gen, beispielsweise Vinyl(meth)acrylat, (Meth)allyl-(meth)acryl-Verbindungen, beispielsweise (Meth)-allyl(meth)-acrylat, mit 1 bis 30 Mol Ethylenoxid pro Hydroxylgruppe ethoxyliertes (Meth)allyl(meth)acrylat oder Di(meth)allylester von Polycarbonsäuren, beispielsweise Di(meth)allylmaleat, Di(meth)allyfumarat, Di(meth)-allylsuccinat oder Di(meth)allylterephthalat.

Des weiteren als weitere Vernetzer a2) bevorzugt sind Verbindungen mit 3 oder mehr ethylenisch ungesättigten Gruppen, wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri-(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi-(meth)acrylat, 3-Allyloxy-1,2-propandioldi(meth)-acrylat, Tri(meth)allyl-cyanurat, Tri(meth)allylisocyanurat, Pentaerythrittetra-(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri-(meth)allylamin, Di(meth)allylalkylamine, beispielsweise Di(meth)allylmethylamin, Tri(meth)allylphosphat Tetra(meth)allylethylendiamin, Poly(meth)allylester, Tetra(meth)allyloxiethan oder Tetra(meth)allylammoniumhalide. Des weiteren als weitere Vernetzer a2) bevorzugte Verbindungen sind Verbindungen mit 4, 5 oder mehr ethylenisch ungesättigten Gruppen, wie beispielsweise Pentaerithrit-Tetra(meth)acrylat, Pentaerythrit-Penta(meth)acrylat oder Pentaerythrit-Hexa(meth)acrylat, wobei Pentaeritrit-Pentaacrylat besonders bevorzugt ist.

Unter diesen Verbindungen insbesondere bevorzugt sind Triethylenglykoldimethacrylat, Tripropylenglykoldiacrylat, Trimethylolpropantriacrylat, Bis(2-Methacryloxyethyl)-N,N'-nonylenbiscarbamat oder Mischungen aus mindestens zwei dieser Vernetzer.

Weitere bevorzugte, mindestens zwei ethylenisch ungesättigte Gruppen aufweisende weitere Vernetzer a2), die gegebenenfalls in Kombinationen mit den vorstehend bereits beschriebenen Vernetzern eingesetzt werden können, sind vorzugsweise aliphatische, difunktionelle Urethan-Oligomer-(Meth)Acrylate wie Urethan-dimethacrylate, Polyester-Urethan-Oligomere-(Meth)Acrylate, Polyether-Urethan-Oligomer-(Meth)Acrylate, Polybutadien-Urethan-Oligomer-(Meth)-Acrylate oder Polycarbonat-Urethan-Oligomer-(Meth)Acrylate, wobei Urethan-Oligomer-(Meth)Acrylate, Polyester-Urethan-Oligomere-(Meth)Acry-late und Mischungen aus Urethan-Oligomer-(Meth)Acrylaten und Polyester-Urethan-Oligomere-(Meth)Acrylaten besonders bevorzugt sind. Ganz besonders bevorzugt sind Polyester-Urethan-Oligomere-(Meth)Acrylate. Unter einem Polyester-Urethan-Oligomer-(Meth)Acrylat wird beispielsweise eine Verbindung verstanden, die mindestens Polyester-, Urethan- und zwei oder mehr (Meth)Acrylat-Gruppierungen enthält. Derartige Urethan-Oligomer-(Meth)Acrylate sind zugänglich, indem ein Diol oder ein Polyester-, Polyether-, Polybutadien-und/oder Polycarbonat-Diol mit einem aliphatischen, cycloaliphatischen und/oder aromatischen Diisocyanat, beispielsweise 1,6-Hexamethylendiisocyanat (HDI), 2,4,4-Trimethyl-hexa-methylen-1,6-diisocyanat (TMDI), Tetramethylendiisocyanat, Isophorondiisocyanat, 4,4'-Dicyclohexyl-methandiisocyanat, 1,4-Phenylendiiso-cyanat, 2,6- und 2,4-Toluoldiisocyanat, 1,5-Naphthylendiisocyanat, 2,4'- und 4,4'-Diphenylmethandiisocyanat (Diisocyanatkomponente) unter Amin oder Zinnkatalyse umgesetzt wird (siehe C. Hepburn, "Polyurethane Elastomers", 2nd Ed, Elsevier Applied Science, London and New York, 1992).

Wird hierbei mit einem molaren Überschuss an Diolkomponente im Vergleich zur Diisocyanatkomponente gearbeitet, bleiben endständige OH-Gruppen übrig, die mit einer ethylenisch ungesättigten Säure, wie Acrylsäure oder Methacrylsäure bzw. einem ihrer Derivate, verestert werden. Wird mit einem molaren Überschuss an Diisocyanatkomponente im Vergleich zur Diolkomponente gearbeitet, bleiben endständige Isocyanatgruppen übrig, die mit einem Hydroxyalkyl- und/oder Hydroxyaryl(meth)acrylat und/oder -di(meth)acrylat und/oder -tri(meth)acrylat, wie beispielsweise 2-Hydroxyethylacrylat (HEA), 2-Hydroxyethylmethacrylat (HEMA), 3-Hydroxypropylmethacrylat (HPMA), 3-Hydroxypropylacrylat (HPA) Glycerindimethacrylat und/oder Glycerindiacrylat, umgesetzt werden.

Die als Vernetzer a2) vorliegenden Urethan-Oligomer-(Meth)Acrylate bzw. die Polyester-, Polyether-, Polybutadien- oder Polycarbonat-Urethan-Oligomer-(Meth)Acrylate weisen vorzugsweise ein zahlenmittleres Molekulargewicht zwischen 200 und 10000, besonders bevorzugt zwischen 300 und 8000, und darüber hinaus bevorzugt zwischen 350 und 7000, darüber hinaus noch mehr bevorzugt zwischen 400 und 6000 und am meisten bevorzugt zwischen 400 und 5000 g/mol auf. Das Molekulargewicht wird mittels GPC gegen einen Styrolstandard ermittelt.

Käufliche und erfindungsgemäß geeignete, difunktionelle Urethan(Meth)Acrylate sind beispielsweise Genomer 4297, Genomer 4269, Genomer 4215, Genomer 4246 (Fa. Rahn AG, Zürich), Ebecryl 230, Ebecryl 270, Ebecryl 930, (UCB Chemicals, Kerpen), BR-304, BR-374, BR-3731, BR-582E, BR-7432, BR-204 (Bomar Specialities Co., Winsted), CN9002, CN9004, CN9007, CN9178, CN940, CN9788, CN9893, CN959 (Fa. Sartomer, Exton, USA) oder difunktionelle, auf Polyestern basierende Urethan-(Meth)Acrylate, wie sie beispielsweise von der Rahn AG, Zürich, unter den Produktbezeichnungen Genomer 4205 oder Genomer 4215 oder von der Firma Sartomer unter den Produktbezeichnungen CN962, CN964, CN965 oder CN984 erhalten werden können.

Vorzugsweise enthält die erfindungsgemäße Zusammensetzung im Wesentlichen keine Epoxidharze. "Im Wesentlichen" bedeutet im Rahmen der Erfindung, dass Epoxidharze im Rahmen technischer Verunreinigungen enthalten sein können. Bevorzugt sind weniger als 1 Gew.-%, besonders bevorzugt weniger als 0,5 Gew.-% und besonders bevorzugt weniger als 0,1 Gew.-% Epoxidharze enthalten, jeweils bezogen auf das Gesamtgewicht der Zusammensetzung. Ganz besonders bevorzugt ist gar kein Epoxidharz in der Zusammensetzung enthalten. Zu den Epoxidharzen zählen insbesondere acrylierte Epoxidharze.

Bei dem Photoinitiator a3) handelt es sich vorzugsweise um unter der Einwirkung elektromagnetischer Strahlung, insbesondere unter der Einwirkung von UV-Licht Radikale bildende Initiatoren. Vorteilhafterweise weist der Initiator eine oder mehrere elektronenziehende Gruppen auf, insbesondere eine Carbonylgruppe, bevorzugt eine Ketogruppe mit einem substituierten oder nicht substituierten aromatischen Ringsystem. Beispiele für geeignete Photoinitiatoren, sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethyl-amino)ethylsulfon, N-(4-Sulfonylazidophenyl)maleinimid, N-Acetyl-4-sulfo-nylazidoanilin, 4-Sulfonylazidoanilin, 4-Azidoanilin, 4-Azidophenacylbromid, p-Azidobenzoesäure, 2,6-Bis(p-azidobenzyliden)-cyclohexanon und 2,6-Bis-(p-azidobenzyliden)-4-methylcyclohexanon. Weitere, erfindungsgemäß bevorzugte Photoinitiatoren umfassen Benzoyl-Derivate wie 2-Hydroxy-2-methylpropiophenon, Benzoylisopropanol, Alkylbenzoylcarbonsäureester, Diphenyl-(2,4,6-trimethyl-benzoyl)phosphinoxid und Ethyl-(2,4,6-trimethylbenzoyl)-phenylphosphinat. Die Alkylbenzoylcarbonsäureester umfassen vorzugsweise Ester der Ameisensäure. Die Alkylgruppe des Esters umfasst vorzugsweise 1 bis 4 C-Atome, wobei die Methylgruppe bevorzugt ist. Ein besonders bevorzugter Alkylbenzoylcarbonsäureester ist Methylbenzoylformiat. Weitere geeignete Photoinitiatoren sind beispielsweise in der US-A-4,792,632 und der US-A-4,737,593 beschriebenen. Erfindungsgemäß ganz besonders bevorzugt ist der Einsatz von Ethyl-(2,4,6-trimethylbenzoyl)-phenylphosphinat, Methylbenzoylformiat, Phenylketon, insbesondere Ethyl-(2,4,6-trimethylbenzoyl)-phenylphosphinat oder Methylbenzoylformiat, Benzophenon-1, Diphenyl(2,4,6-trimethylbenzoyl)phosphinoxid, 2-Hydroxy-2-methylpropiophenon und Mischungen aus mindestens zwei dieser Initiatoren. Ethyl-(2,4,6-trimethylbenzoyl)-phenylphosphinat stellt ein überragendenden Photoinitiator a3) im Sinne der Erfindung dar.

Vorzugsweise ist der Photoinitiator nicht ausgewählt aus einer Mischung aus 2-Hydroxy-2-methyl-1-methylpropan-1-on, insbesondere eines Acetophenon-Derivats, und Diphenyl(2,4,6-trimethylbenzoyl)phosphinoxid, insbesondere eines Acylphosphinoxids.

Der Photoinitiator a3) kann in einem Anteil von 1 bis 5 Gew.-%, vorzugsweise 1 bis 4 Gew.-% enthalten sein.

Die erfindungsgemäße Zusammensetzung kann neben dem vorstehend beschriebenen durch elektromagnetische Strahlung härtbaren Mehrkomponentensystem a) als Komponente b) 0 bis 4 Gew.-% eines Disaccharidesters zur Erhöhung von Glanz und/oder der Haftung der Lackierung. Als Disaccharidester sind dabei Sucroseester, insbesondere Sucroseester aus Sucrose und einer armatischen Carbonsäure besonders bevorzugt, wobei der Einsatz von Sucrosebenzoat als Disaccharidester am meisten bevorzugt ist.

Die aromatische Carbonsäure verursacht vermutlich einen negativen mesomeren Effekt an dem Sauerstoffatom der Carbonylgruppe, so dass stärker ausgebildete Wasserstoffbrückenbindungen beispielsweise zu Acetylgruppen der Monomeren und Oligomeren ausgebildet werden können. Vorzugsweise ist die Komponente b) in einem Anteil von 0 bis 3 Gew.-%, bevorzugt 0 bis 2 Gew.-%, besonders bevorzugt 0 bis 1 Gew.-% und insbesondere gar nicht enthalten.

Bei Sucrosebenzoat handelt es sich vorzugsweise um einen Ester, bei denen mindestens fünf, vorzugsweise mindestens 6, noch mehr bevorzugt mindestens 7 und am meisten alle 8 OH-Gruppen in der Sucrose-Einheit mit Benzoesäure verestert sind.

In einer Ausführungsform werden Disaccharidester, welche als Filmbildner fungieren, mit Ausnahme von Saccharoseacetatisobutyrat eingesetzt. Besonders bevorzugt sind Disaccharidester, wobei weiterhin mindestens eine der folgenden Komponenten (bevorzugt alle der folgenden Komponenten) Saccharosepalmitat, Saccharosedistearat, Saccharoselaurat, Sucrosemyristat, Sucrosetristearat und Sucrosetetrastearat ausgenommen sind.

Ein Filmbildner ist nach DIN 55 945 (12/1988) ein Bestandteil, der für das Zustandekommen der Beschichtung wesentlich ist (vgl. Römpp ChemieLexikon, 10. Auflage 1996, Georg Thieme Verlag Stuttgart, ISBN 3-13-107830-8, Stichworte "Filmbildner", "Anstrich" und "Anstrichstoffe"). Die Filmbildung stellt insbesondere den Übergang eines aufgetragenen Beschichtungsstoffes vom flüssigen in den festen Zustand dar. Die Filmbildung erfolgt durch Trocknung und/oder Härtung. Beide Vorgänge können gleichzeitig ablaufen (DIN EN ISO 4618:2006, Stichwort "Filmbildung").

Neben den vorstehend beschriebenen Komponenten a) und b) kann die erfindungsgemäße Zusammensetzung als weitere Komponente c) ein Lösungsmittel oder ein Lösungsmittelgemisch beinhalten, wobei die Zusammensetzung vorzugsweise 0 bis 25 Gew.-% Lösungsmittel beinhaltet. Bevorzugt beträgt der Lösungsmittel-Anteil 1 bis 25 Gew.-%, besonders bevorzugt 2 bis 25 Gew.-% und ganz besonders bevorzugt 5 bis 20 Gew.-% und insbesondere 5 bis 15 Gew.-%.

Als Lösungsmittel kommen hierbei insbesondere Ester, wie etwa Methylacetat, Ethylacetat, Isopropylacetat oder Butylacetat, Alkohole wie etwa Methanol, Ethanol, Trimethylolpropan oder Isopropanol, Ketone wie Aceton, Methylethylketon oder Methylisobutylketon, oder aber Mischungen aus mindestens zwei dieser Lösungsmittel in Betracht, wobei der Einsatz eines Lösungsmittels ausgewählt aus der Gruppe bestehend aus Ethylacetat, Butylacetat, Trimethylolpropan, Isopropylalkohol, bevorzugt Ethylacetat, Butylacetat und Isopropylalkohol, und einer Mischung aus mindestens zwei dieser Lösungsmittel ganz besonders bevorzugt ist.

Schließlich kann die erfindungsgemäße Zusammensetzung als weitere Komponente auch von den Komponenten a) bis c) verschiedene Additive d) beinhalten, wobei hierbei insbesondere diejenigen Additive in Betracht kommen, die üblicherweise in kosmetischen Zusammensetzungen, insbesondere in einem Nagellacksystem, enthalten sind. Beispielhaft zu nennen sind hier vor allem d1)
sekundäre filmbildende Additive neben der als Filmbildner fungierenden Komponente b),
d2) farbgebende Mittel,
d3) Weichmacher,
d4) Antioxidationsmittel,
d5) Emulgatoren.

Als filmbildende Additive d1) kommen, neben der als Filmbildner fungierenden Komponente b), insbesondere Cellulose-Derivate wie beispielsweise Nitrocellulose, Methylcellulose, Ethylcellulose, Propylcellulose, Butylcellulose, Cellulosephthalat, Celluloseacetatpropionat, Celluloseacetatbutyrat, Celluloseacetat oder Hydroxypropylcellulose, Butyralpolyvinylverbindungen, Alkydharze, Harze, die bei der Kondensation von Formaldehyd mit Arylsulfonamid entstehen, Polyester, Polyurethane, Polyesterpolyurethane, Polyetherpolyurethane, radikalisch hergestellte Polymeren wie etwa Polyacrylate oder Copolymere aus Acrylsäure und anderen ethylenisch ungesättigten Monomeren, wie etwa Copolymere aus Acrylsäure und Styrol, Anhydride wie Phthalsäureanhydrid oder Trimellitsäureanhydrid, oder aber Mischungen aus mindestens zwei dieser filmbildenden Substanzen in Betracht.

Die filmbildenden Additive d1) sind vorzugsweise in einem Anteil von 0,3 bis 11,5 Gew.-%, besonders bevorzugt 0,4 bis 10 Gew.-% in der Zusammensetzung enthalten.

Die Cellulose-Derivate und Acrylat-Copolymere sind bevorzugte filmbildende Additive d1). In diesem Zusammenhang ist es erfindungsgemäß besonders bevorzugt, dass die Zusammensetzung 2 bis 4 Gew.-% Nitrozellulose als filmbildendes Additiv gemäß Komponente d1) beinhaltet.

Als farbgebende Mittel d2) kommen alle dem Fachmann für den Einsatz in Nagellacken bekannten Farbstoffe oder Pigmente in Betracht, wobei der Unterschied zwischen einem Farbstoff und einem Pigment im Sinne der vorliegenden Erfindung darin besteht, dass das Pigment im Gegensatz zum Farbstoff in der erfindungsgemäßen Zusammensetzung nicht löslich ist. Erfindungsgemäß besonders bevorzugt ist der Einsatz organischer oder anorganischer Pigmente, wobei hier diejenigen organischen oder anorganischen Pigmente besonders bevorzugt sind, die in der DE 10 2005 063 061 A1 als bevorzugte Pigmente genannt werden. Ganz besonders bevorzugt sind in der erfindungsgemäßen Zusammensetzung anorganische Pigmente enthalten, wobei erfindungsgemäß geeignete anorganische Pigmente und deren Herstellweisen G. Buxbaum; "Industrial Inorganic Pigments", 1. Aufl., S. 85-107; VCH Verlagsgesellschaft mbH, Weinheim, 1993, G. Buxbaum; "Industrial Inorganic Pigments", 1. Aufl., S. 114-117; VCH Verlagsgesellschaft mbH, Weinheim, 1993 sowie G. Buxbaum; "Induslrial Inorganic Pigmente", 1. Aufl., S. 124-131; VCH Verlagsgesellschaft mbH, Weinheim, 1993 entnommen werden können. Gegebenenfalls können die Pigmente auch silanisiert sein, wie dies in der DE 10 2005 063 061 A1 beschrieben ist.

Sofern farbgebende Mittel d2) enthalten sein sollen, sind Anteile von 0,3 bis 15 Gew.-% üblich, wobei 0,5 bis 7,5 Gew.-% bevorzugt sind.

Als Weichmacher d3) kommen insbesondere Verbindungen ausgewählt aus der Gruppe bestehend aus Trikresylphosphat, Benzylbenzoat, Tributylphosphat, Butylacetylricinoleat, Glycerylacetylricinoleat, Dibutylphthalat, Butylglykolat, Dioctylphthalat, Butylstearat, Tributoxyethylphosphat, Triphenylphosphat, Triethylcitrat, Tributylcitrat, Tributylacetylcitrat, 2-Triethylhexylacetylcitrat, Dibutyltartrat, Dimethoxyethylphthalat, Diisobutylphthalat, Diamylphthalat, Campher, Glyceryltriacetat und deren Gemische in Betracht, wobei der Einsatz von Tributylacetylcitrat ganz besonders bevorzugt ist.

Üblicherweise sind 0,5 bis 3 Gew.-% an Weichmachern in der erfindungsgemäßen Zusammensetzung enthalten.

Als Antioxidationsmittel d4) kommen insbesondere solche des Phenoltyps, wie etwa Propyl-, Octyl- und Dodecylester der Gallussäure, p-Hydroxyanisol, Butyl-hydroxyanisol oder Butyl-hydroxytoluol, aber auch Verbindungen wie Tocopherol und Ascorbylpalmitat in Betracht, wobei der Einsatz von Butylhydroxytoluol besonders bevorzugt ist. Das Antioxidationsmittel d4) kann in einer Menge von 0 bis 2, vorzugsweise 0 bis 1 Gew.-% enthalten sein.

Als Emulgatoren d5) können alle dem Fachmann für einen Einsatz in Nagellacksystemen bekannten Emulgatoren eingesetzt werden, wie etwa PEG-9-Stearat, PEG-8-Distearat, PEG-20-Stearat, PEG-8-Stearat, PEG-8-Oleat, PEG-25-Glyceryltrioleat, PEG-40-Sorbitanlanolat, PEG-15-Glycerylricinoleat, PEG-20- Glycerylstearat, PEG-20-Glycerylisostearat, PEG-20-Glyceryloleat, PEG-20-Stearat, PEG-20-Methylglucosesesquistearat, PEG-30-Glyceryl-isostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat, Butyloctanol, Butyldecanol, Hexyloctanol, Hexyldecanol, Octyldodecanol, Behenylalkohol, Cetearylalkohol, Stearylalkohol oder Lanolinalkohole. Als weiteres Beispiel eines geeigneten Emulgators sei insbesondere Silika-Dimethylsilylat genannt.

Neben den vorstehend genannten Additiven d1) bis d5) können auch andere dem Fachmann für einen Einsatz in Nagellacksystemen bekannte Additive in der erfindungsgemäßen Zusammensetzung enthalten sein, wie etwa Viskositätsregulierer, Glanzmittel, Fließmittel, oder Aufheller. Der Fachmann wird dabei die Menge dieser Additive und der vorstehend beschriebenen Additive d1) bis d5) so bemessen, dass die vorteilhaften Eigenschaften der erfindungsgemäßen Zusammensetzung (schnelles und kontrollierbares Aushärten, hohe Kratzfestigkeit und leichte Ablösbarkeit) erhalten bleiben.

Die Viskosität der erfindungsgemäßen Zusammensetzung beträgt bei 23 °C vorzugsweise 2100 bis 2900 mPa·s (Rotationsviskosimeter Brookfield Digital, Modell DV-II+ Pro, RV Spindel 0,5). Hierdurch wird eine dem Fachmann geläufige Nagellack-artige Konsistenz erhalten.

Gemäß einer ersten besonderen Ausführungsform der erfndungsgemäßen Zusammensetzung beinhaltet diese 75 bis 95 Gew.-%, besonders bevorzugt 80 bis 90 Gew.-% des Mehrkomponentensystems a). In diesem Zusammenhang ist es besonders bevorzugt, dass die Zusammensetzung 10 bis50 Gew.-%, besonders bevorzugt 20 bis 35 Gew.-% der Komponente a1) und 25 bis 75 Gew.-%, besonders bevorzugt 45 bis 65 Gew.-% der Komponente a2) beinhaltet, jeweils bezogen auf das Gesamtgewicht der Zusammensetzung. Am meisten bevorzugt ist es in diesem Zusammenhang, dass die Zusammensetzung 15 bis 30 Gew.-%, besonders bevorzugt 20 bis 25 Gew.-% Hydroxyethylmethacyrlat als Komponente a1) und 50 bis 70 Gew.-%, besonders bevorzugt 55 bis 65 Gew.-% der Komponente a2) beinhaltet, jeweils bezogen auf das Gesamtgewicht der Zusammensetzung. Der Lösemittelanteil beträgt vorzugsweise 1 bis 15 Gew.-%, bevorzugt 5 bis 15 Gew.-%. Das filmbildende Additiv d1) ist vorzugsweise in einem Bereich von 1 bis 3 Gew.-% enthalten. Die Zusammensetzung enthält vorzugsweise 0,02 bis 0,2 Gew.-% eines Antioxidationsmittels d4). Weiterhin ist vorzugsweise 1,5 bis 3 Gew.-% eines Photoinitiators a3) enthalten. Die Zusammensetzungen der ersten besonderen Ausführungsform können Pigmente enthalten, wobei deren Anteil vorzugsweise 0 bis 0,5 Gew.-%, bevorzugt 0 bis 0,1 Gew.-% und besonders bevorzugt 0 bis 0,01 Gew.-% betragen kann.

Eine solche Zusammensetzung eignet sich besonders vorteilhaft als unmittelbar auf die Oberfläche eines Finger- oder Zehennagels auftragbare Basisschicht in dem nachfolgend noch beschriebenen erfindungsgemäßen Verfahren zur kosmetischen Behandlung eines Finger- oder Zehennagels.

Eine solche Zusammensetzung eignet sich zudem besonders vorteilhaft als auf eine farbgebende Schicht folgende Glanzschicht in dem nachfolgend noch beschriebenen erfindungsgemäßen Verfahren zur kosmetischen Behandlung eines Finger- oder Zehennagels.

Gemäß einer zweiten besonderen Ausführungsform der erfindungsgemäßen Zusammensetzung beinhaltet diese 65 bis 85 Gew.-%, besonders bevorzugt 70 bis 80 Gew.-% des Mehrkomponentensystems a) sowie 1 bis 20 Gew.%, besonders bevorzugt 2 bis 10 Gew.-% eines farbgebenden Mittels als Komponente d2). In diesem Zusammenhang ist es besonders bevorzugt, dass die Zusammensetzung 10 bis 60 Gew.-%, besonders bevorzugt 25 bis 40 Gew.-% der Komponente a1), 25bis 75 Gew.-%, besonders bevorzugt 40 bis 60 Gew.-% der Komponente a2) und 2 bis 10 Gew.-%, besonders bevorzugt 3 bis 8 Gew.-% und ganz besonders bevorzugt 3,5 bis 6 Gew.-% eines farbgebenden Mittels als Komponente d2) beinhaltet, jeweils bezogen auf das Gesamtgewicht der Zusammensetzung. Am meisten bevorzugt ist es in diesem Zusammenhang, dass die Zusammensetzung 20 bis 40 Gew.-%, besonders bevorzugt 25 bis 35 Gew.-% Hydroxyethylmethacyrlat als Komponente a1), 35 bis 55 Gew.-%, besonders bevorzugt 40 bis 50 Gew.-% der Komponente a2), 0,1 bis 2 Gew.-%, besonders bevorzugt 0,2 bis 0,6 Gew.-% Butylhydroxytoluol als Komponente d4) und 2 bis 10 Gew.-%, besonders bevorzugt 3 bis 8 Gew.-% und ganz besonders bevorzugt 3,5 bis 6 Gew.-% eines farbgebenden Mittels als Komponente d2) beinhaltet, jeweils bezogen auf das Gesamtgewicht der Zusammensetzung. Der Lösemittelanteil beträgt vorzugsweise 2 bis 30 Gew.-% und bevorzugt 5 bis 20 Gew.-%. Das filmbildende Additiv d1) ist vorzugsweise in einem Bereich von 1 bis 5 Gew.-%, vorzugsweise 1 bis 4 Gew.-% enthalten. Weiterhin ist es ganz besonders bevorzugt, dass die Zusammensetzung 1,5 bis 4 Gew.-% eines Photoinitiators a3) enthält. Weiterhin ist es ganz besonders bevorzugt, dass 1 bis 3 Gew.-% eines Emulgators d5) enthalten sind.

Eine solche Zusammensetzung eignet sich besonders vorteilhaft als auf die Basisschicht folgende farbgebende Schicht in dem nachfolgend noch beschriebenen erfindungsgemäßen Verfahren zur kosmetischen Behandlung eines Finger- oder Zehennagels.

Einen Beitrag zur Lösung der eingangs genannten Aufgaben leistet auch ein System, vorzugsweise ein Kit, zur Behandlung eines Finger- oder Zehennagels, umfassend die Systemkomponenten:
S1) eine erfindungsgemäße Zusammensetzung gemäß der vorstehend beschriebenen ersten besonderen Ausführungsform,
S2) eine erfindungsgemäße Zusammensetzung gemäß der vorstehend beschriebenen zweiten besonderen Ausführungsform, sowie
S3) eine erfindungsgemäße Zusammensetzung gemäß der vorstehend beschriebenen ersten besonderen Ausführungsform.

Die im System eingesetzte Systemkomponente S1 kann gleich oder verschieden sein zu der Systemkomponente S3. Vorzugsweise sind die Systemkomponenten S1 und S3 identisch. Demzufolge ist ein System bevorzugt, umfassend die Systemkomponenten:
S1) eine erfindungsgemäße Zusammensetzung gemäß der vorstehend beschriebenen ersten besonderen Ausführungsform, sowie
S2) eine erfindungsgemäße Zusammensetzung gemäß der vorstehend beschriebenen zweiten besonderen Ausführungsform.

Ein solches System eignet sich insbesondere zur kosmetischen Behandlung eines Finger- oder Zehennagels durch das nachfolgend beschriebene erfindungsgemäße Verfahren.

Einen Beitrag zur Lösung der eingangs genannten Aufgaben leistet auch ein Verfahren zur kosmetischen Behandlung eines Finger- oder Zehennagels, beinhaltend die Verfahrensschritte:
I) Auftragen einer Schicht einer Zusammensetzung gemäß der vorstehend beschriebenen ersten besonderen Ausführungsform auf die Oberfläche eines Finger- oder Zehennagels und anschließendes Aushärten dieser Schicht durch elektromagnetische Strahlung, besonders bevorzugt durch UV-Strahlung, unter Erhalt einer ausgehärteten Grundschicht;
II) Auftragen einer Schicht einer Zusammensetzung gemäß der vorstehend beschriebenen zweiten besonderen Ausführungsform auf die im Verfahrensschritt I) erhaltene ausgehärtete Schicht und anschließendes Aushärten dieser Schicht durch elektromagnetische Strahlung, besonders bevorzugt durch UV-Strahlung, unter Erhalt einer ausgehärteten Farbschicht;
III) Auftragen einer Schicht einer Zusammensetzung gemäß der vorstehend beschriebenen ersten besonderen Ausführungsform auf die im Verfahrensschritt II) erhaltene ausgehärtete Farbschicht und anschließendes Aushärten dieser Schicht durch elektromagnetische Strahlung, besonders bevorzugt durch UV-Strahlung, unter Erhalt einer ausgehärteten Glanzschicht.

Die in den Verfahrensschritten I und III eingesetzten Zusammensetzungen gemäß der ersten besonderen Ausführungsform können gleich oder verschieden sein. Vorzugsweise sind sie gleich.

Im ersten Schritt des erfindungsgemäßen Verfahrens wird zunächst eine Grundschicht auf der Basis der ersten besonderen Ausführungsform der erfindungsgemäßen Zusammensetzung auf die Oberfläche eines Finger- oder Zehennagels aufgebracht, wobei das Aufbringen vorzugsweise durch ein Applizieren mit Hilfe eines Pinsels erfolgt, wobei ein feiner Pinsel, insbesondere ein Pinsel mit einer flachen Kante oder ein Pinsel, der mittels leichtem Druck flach gedrückt werden kann, besonders bevorzugt ist. Üblicherweise wird die Zusammensetzung in Form einer 0,01-0,16 mm, vorzugsweise einer 0,05 bis 0,1 mm dicken Schicht aufgetragen. Anschließend wird die Schicht durch die Einwirkung elektromagnetischer Strahlung, besonders bevorzugt durch UV-Strahlung, ausgehärtet, wobei hier insbesondere UV-Lampen, etwa solche, die in der DE 20 2005 018 552 U1 oder der DE 20 2004 008 982 U1 beschrieben werden, zum Einsatz kommen können.

Im zweiten Verfahrensschritt wird sodann auf die ausgehärtete Grundschicht, welche im ersten Verfahrensschritt erhalten wurde, eine Farbschicht auf der Basis der zweiten besonderen Ausführungsform der erfindungsgemäßen Zusammensetzung aufgebracht und anschließend ausgehärtet, wobei das Aufbringen und Aushärten vorzugsweise auf die gleiche Art und Weise erfolgt, in der auch die erste Schicht aufgebracht und ausgehärtet wurde. Gemäß einer besonderen Ausführungsform des erfindungsgemäßen Verfahrens kann der Verfahrensschritt II) mindestens zweimal durchgeführt werden.

Im dritten Verfahrensschritt wird sodann auf die (letzte) ausgehärtete Farbschicht, welche im zweiten Verfahrensschritt erhalten wurde, eine finale Glanzschicht auf der Basis der ersten besonderen Ausführungsform der erfindungsgemäßen Zusammensetzung aufgebracht und anschließend ausgehärtet, wobei das Aufbringen und Aushärten vorzugsweise auf die gleiche Art und Weise erfolgt, in der auch die erste und die zweite Schicht aufgebracht und ausgehärtet wurden.

Aus dem erfindungsgemäßen Verfahren resultieren ausgehärtete Lackfilme, die wie eine Art Klebefolie abziehbar und rückstandslos entfernt werden können.

Ein weiterer Vorteil der vorliegenden Erfindung ist darin zu sehen, dass die Basis- und die Glanzschicht die gleiche Zusammensetzung aufweisen können.

Gemäß einer besonderen Ausführungsform des erfindungsgemäßen Verfahrens kann im Anschluss an den Verfahrensschritt III) die äußere ausgehärtete Glanzschicht mit einer Reinigungszusammensetzung behandelt werden.

Die Erfindung wird nun anhand nicht limitierender Beispiele näher erläutert.

### Beispiel 1

Beispiel einer Zusammensetzung gemäß der ersten besonderen Ausführungsform

| Verbindung | Komponente | Menge [Gew.-%] |
|---|---|---|
| ethylenisch ungesättigte Gruppe aufweisendes Monomer | a1) | 21,5 |
| Vernetzer mit mindestens zwei ethylenisch ungesättigten Gruppen | a2) | 63,5 |
| Benzoyl-Derivat als Photoinitiator | a3) | 2,3 |
| Lösemittel | c) | 9,0 |
| Cellulose-Derivat und Acrylat-Copolymer als filmbildendes Additiv | d1) | 2,5 |
| Weichmacher auf Citrat-Basis | d3) | 1,0 |
| Antioxidationsmittel des Phenoltyps | d4) | 0,2 |

Die vorstehende Zusammensetzung kann als Grundschicht oder als Glanzschicht für das erfindungsgemäße Nagellacksystem eingesetzt werden.

### Beispiel 2

Beispiel einer Zusammensetzung gemäß der zweiten besonderen Ausführungsform:

| Verbindung | Komponent e | Menge [Gew.-%] |
|---|---|---|
| ethylenisch ungesättigte Gruppe aufweisendes Monomer | a1) | 29,5 |
| Vernetzer mit mindestens zwei ethylenisch ungesättigten Gruppen | a2) | 45,9 |
| Benzoyl-Derivat als Photoinitiator | a3) | 3,0 |
| Lösemittel | c) | 11,3 |
| Cellulose-Derivat und Acrylat-Copolymer als filmbildendes Additiv | d1) | 2,5 |
| Pigmente | d2) | 5,1 |
| Weichmacher auf Citrat-Basis | d3) | 1,2 |
| Antioxidationsmittel des Phenoltyps | d4) | 0,4 |
| Emulgator | d5) | 1,5 |

Die vorstehende Zusammensetzung kann als Farbschicht für das erfindungsgemäße Nagellacksystem eingesetzt werden.

### Beispiel 3

Beispiel einer Zusammensetzung gemäß der zweiten besonderen Ausführungsform

| Verbindung | Komponente | Menge [Gew.-%] |
|---|---|---|
| ethylenisch ungesättigte Gruppe aufweisendes Monomer | a1) | 28,2 |
| Vernetzer mit mindestens zwei ethylenisch ungesättigten Gruppen | a2) | 40,8 |
| Benzoyl-Derivat als Photoinitiator | a3) | 3,1 |
| Lösemittel | c) | 9,3 |
| Cellulose-Derivat und Acrylat-Copolymer als filmbildendes Additiv | d1) | 1,8 |
| Pigmente | d2) | 14,6 |
| Weichmacher auf Citrat-Basis | d3) | 0,7 |
| Antioxidationsmittel des Phenoltyps | d4) | 0,4 |
| Emulgator | d5) | 1,1 |

Die vorstehende Zusammensetzung kann als Farbschicht für das erfindungsgemäße Nagellacksystem eingesetzt werden.

### Beispiel 4

Beispiel einer Zusammensetzung gemäß der ersten besonderen Ausführungsform

| Verbindung | Komponente | Menge [Gew.-%] |
|---|---|---|
| ethylenisch ungesättigte Gruppe aufweisendes Monomer | a1) | 28,1 |
| Vernetzer mit mindestens zwei ethylenisch ungesättigten Gruppen | a2) | 58,0 |
| Benzoyl-Derivat als Photoinitiator | a3) | 1,4 |
| Lösemittel | c) | 9,2 |
| Cellulose-Derivat und Acrylat-Copolymer als filmbildendes Additiv | d1) | 1,8 |
| Weichmacher auf Citrat-Basis | d3) | 1,0 |
| Antioxidationsmittel des Phenoltyps | d4) | 0,5 |

Die vorstehende Zusammensetzung kann als Grundschicht oder als Glanzschicht für das erfindungsgemäße Nagellacksystem eingesetzt werden.

Die Systeme aus Grund-, Farb- und Glanzschicht ließ sich nach Aushärtung der Schichten (ausgehärteter Lackfilm) wie eine Art Klebefolie abziehen und rückstandslos entfernen.

## Patentansprüche

1. Zusammensetzung, beinhaltend die Komponenten
a) 25 bis 95 Gew.-% eines durch elektromagnetische Strahlung härtbaren Mehrkomponentensystems umfassend a1
) mindestens ein eine ethylenisch ungesättigte Gruppe aufweisendes Monomer,
a2) mindestens ein aliphatisches, difunktionelles Polyester-Urethan-Oligomer-(Meth)Acrylat als mindestens zwei ethylenisch ungesättigte Gruppen aufweisender Vernetzer, sowie
a3) mindestens einen Photoinitiator,
b) 0 bis 4 Gew.-% eines Disaccharidesters,
c) 0 bis 30 Gew.-% Lösungsmittel, und
d) 0 bis 50 Gew.-% von den Komponenten a) bis c) verschiedene Additive,
wobei die Mengenangaben jeweils auf das Gesamtgewicht der Zusammensetzung bezogen sind und wobei die Summe der Komponenten a) bis d) 100 Gew.-% beträgt, und
wobei die Zusammensetzung 0,3 bis 5 Gew.-% Nitrozellulose als filmbildendes Additiv gemäß Komponente d) beinhaltet.

2. Die Zusammensetzung nach Anspruch 1, wobei das Mehrkomponentensystem a) ein durch UV-Licht härtbares Mehrkomponentensystem ist.

3. Die Zusammensetzung nach Anspruch 1 oder 2, wobei der Disaccharidester b) Sucrosebenzoat ist.

4. Die Zusammensetzung nach einem der vorhergehenden Ansprüche, wobei die Zusammensetzung 1 bis 25 Gew.-% Lösungsmittel beinhaltet.

5. Die Zusammensetzung nach Anspruch 4, wobei das Lösungsmittel ausgewählt ist aus der Gruppe bestehend aus Ethylacetat, Butylacetat, Trimethylolpropan, Isopropylalkohol und einer Mischung aus mindestens zwei dieser Lösungsmittel.

6. Die Zusammensetzung nach einem der Ansprüche 1 bis 5, wobei die Zusammensetzung 75 bis 95 Gew.-% des Mehrkomponentensystems a) beinhaltet.

7. Die Zusammensetzung nach Anspruch 6, wobei die Zusammensetzung 10 bis 50 Gew.-% der Komponente a1) und 25 bis 75 Gew.-% der Komponente a2) beinhaltet, jeweils bezogen auf das Gesamtgewicht der Zusammensetzung.

8. Die Zusammensetzung nach Anspruch 7, wobei die Zusammensetzung 10 bis 50 Gew.-% Hydroxyethylmethacyrlat als Komponente a1) und 25 bis 75 Gew.-% der Komponente a2) beinhaltet, jeweils bezogen auf das Gesamtgewicht der Zusammensetzung.

9. Die Zusammensetzung nach einem der Ansprüche 1 bis 5, wobei die Zusammensetzung 65 bis 85 Gew.-% des Mehrkomponentensystems a) und 1 bis 20 Gew.% eines farbgebenden Mittels als Komponente d2) beinhaltet.

10. Die Zusammensetzung nach Anspruch 9, wobei die Zusammensetzung 10 bis 60 Gew.-% der Komponente a1), 25 bis 75 Gew.-% der Komponente a2) und 2 bis 10 Gew.-% eines farbgebenden Mittels als Komponente d2) beinhaltet, jeweils bezogen auf das Gesamtgewicht der Zusammensetzung.

11. Die Zusammensetzung nach Anspruch 9 oder 10, wobei die Zusammensetzung 10 bis 60 Gew.-% Hydroxyethylmethacyrlat als Komponente a1), 25 bis 75 Gew.-% der Komponente a2), 0,1 bis 2 Gew.-% Butylhydroxytoluol als Komponente d4) und 2 bis 10 Gew.-% eines farbgebenden Mittels als weitere Komponente d2) beinhaltet, jeweils bezogen auf das Gesamtgewicht der Zusammensetzung.

12. Ein System zur Behandlung eines Finger- oder Zehennagels, umfassend die Systemkomponenten:
S1) eine Zusammensetzung, wie in einem der Ansprüche 6 bis 8 definiert,
S2) eine Zusammensetzung, wie in einem der Ansprüche 9 bis 11 definiert.

13. Ein Verfahren zur kosmetischen Behandlung eines Finger- oder Zehennagels, beinhaltend die Verfahrensschritte:
I) Auftragen einer Schicht einer Zusammensetzung, wie in einem der Ansprüche 6 bis 8 definiert, auf die Oberfläche eines Finger- oder Zehennagels und anschließendes Aushärten dieser Schicht durch elektromagnetische Strahlung unter Erhalt einer ausgehärteten Grundschicht;
II) Auftragen einer Schicht einer Zusammensetzung, wie in einem der Ansprüche 9 bis 11 definiert, auf die im Verfahrensschritt I) erhaltene ausgehärtete Schicht und anschließendes Aushärten dieser Schicht durch elektromagnetische Strahlung unter Erhalt einer ausgehärteten Farbschicht;
III) Auftragen einer Schicht einer Zusammensetzung, wie in einem der Ansprüche 6 bis 8 definiert, auf die im Verfahrensschritt II) erhaltene weitere ausgehärtete Schicht und anschließendes Aushärten dieser Schicht durch elektromagnetische Strahlung unter Erhalt einer ausgehärteten Glanzschicht.

14. Das Verfahren nach Anspruch 13, wobei der Verfahrensschritt II) mindestens zweimal durchgeführt wird.

15. Das Verfahren nach Anspruch 13 oder 14, wobei die elektromagnetische Strahlung UV-Strahlung ist.

16. Das Verfahren nach einem der Ansprüche 13 bis 15, wobei im Anschluss an den Verfahrensschritt III) die im Verfahrensschritt III) erhaltene äußere ausgehärtete Schicht mit einer Reinigungszusammensetzung behandelt wird.

## Claims

1. Composition including the components
a) 25 to 95 wt% of a multicomponent system that is curable by electromagnetic radiation, comprising
a1) at least one monomer having an ethylenically unsaturated group,
a2) at least one aliphatic, difunctional polyester urethane oligomer (meth)acrylate as crosslinker having at least two ethylenically unsaturated groups, and also
a3) at least one photoinitiator,
b) 0 to 4 wt% of a disaccharide ester,
c) 0 to 30 wt% of solvent, and
d) 0 to 50 wt% of additives other than the components a) to c),
wherein the amounts are based in each case on the total weight of the composition and wherein the sum of the components a) to d) is 100 wt%, and
wherein the composition includes 0.3 to 5 wt% of nitrocellulose as film-forming additive according to component d).

2. Composition according to Claim 1, wherein the multicomponent system a) is a multicomponent system that is curable by UV light.

3. Composition according to Claim 1 or 2, wherein the disaccharide ester b) is sucrose benzoate.

4. Composition according to one of the preceding claims, wherein the composition includes 1 to 25 wt% of solvent.

5. Composition according to Claim 4, wherein the solvent is selected from the group consisting of ethyl acetate, butyl acetate, trimethylolpropane, isopropyl alcohol and a mixture of at least two of these solvents.

6. Composition according to one of Claims 1 to 5, wherein the composition includes 75 to 95 wt% of the multicomponent system a).

7. Composition according to Claim 6, wherein the composition includes 10 to 50 wt% of the component a1) and 25 to 75 wt% of the component a2), in each case based on the total weight of the composition.

8. Composition according to Claim 7, wherein the composition includes 10 to 50 wt% of hydroxyethyl methacrylate as component a1) and 25 to 75 wt% of the component a2), in each case based on the total weight of the composition.

9. Composition according to one of Claims 1 to 5, wherein the composition includes 65 to 85 wt% of the multicomponent system a) and 1 to 20 wt% of a colouring agent as component d2).

10. Composition according to Claim 9, wherein the composition includes 10 to 60 wt% of the component a1), 25 to 75 wt% of the component a2) and 2 to 10 wt% of a colouring agent as component d2), in each case based on the total weight of the composition.

11. Composition according to Claim 9 or 10, wherein the composition includes 10 to 60 wt% of hydroxyethyl methacrylate as component a1), 25 to 75 wt% of the component a2), 0.1 to 2 wt% of butylhydroxytoluene as component d4) and 2 to 10 wt% of a colouring agent as further component d2), in each case based on the total weight of the composition.

12. System for treating a fingernail or toenail, comprising the system components:
S1) a composition as defined in one of Claims 6 to 8,
S2) a composition as defined in one of Claims 9 to 11.

13. Method for the cosmetic treatment of a fingernail or toenail, containing the method steps:
I) applying a layer of a composition as defined in one of Claims 6 to 8 to the surface of a fingernail or toenail and subsequently curing this layer by electromagnetic radiation to obtain a cured base layer;
II) applying a layer of a composition as defined in one of Claims 9 to 11 to the cured layer obtained in method step I) and subsequently curing this layer by electromagnetic radiation to obtain a cured colour layer;
III) applying a layer of a composition as defined in one of Claims 6 to 8 to the further cured layer obtained in method step II) and subsequently curing this layer by electromagnetic radiation to obtain a cured gloss layer.

14. Method according to Claim 13, wherein the method step II) is carried out at least twice.

15. Method according to Claim 13 or 14, wherein the electromagnetic radiation is UV radiation.

16. Method according to one of Claims 13 to 15, wherein, subsequent to the method step III), the outer cured layer obtained in method step III) is treated with a cleaning composition.

## Revendications

1. Composition, contenant les composants
a) 25 à 95% en poids d'un système à plusieurs composants durcissable par un rayonnement électromagnétique, comprenant
a1) au moins un monomère présentant un groupe éthyléniquement insaturé,
a2) au moins un (méth)acrylate aliphatique, difonctionnel d'oligomère de polyester-uréthane en tant que réticulant présentant au moins deux groupes éthyléniquement insaturés ainsi que
a3) au moins un photo-initiateur,
b) 0 à 4% en poids d'un ester de disaccharide,
c) 0 à 30% en poids de solvant et
d) 0 à 50% en poids d'additifs différents des composants a) à c),
les indications de quantités se rapportant à chaque fois au poids total de la composition et la somme des composants a) à d) valant 100% en poids et la composition contenant 0,3 à 5% en poids de nitrocellulose en tant qu'additif filmogène selon le composant d).

2. Composition selon la revendication 1, le système à plusieurs composants a) étant un système à plusieurs composants durcissable par la lumière UV.

3. Composition selon la revendication 1 ou 2, l'ester de disaccharide b) étant le benzoate de saccharose.

4. Composition selon l'une quelconque des revendications précédentes, la composition contenant 1 à 25% en poids de solvant.

5. Composition selon la revendication 4, le solvant étant choisi dans le groupe constitué par l'acétate d'éthyle, l'acétate de butyle, le triméthylolpropane, l'alcool isopropylique et un mélange d'au moins deux de ces solvants.

6. Composition selon l'une quelconque des revendications 1 à 5, la composition contenant 75 à 95% en poids du système à plusieurs composants a).

7. Composition selon la revendication 6, la composition contenant 10 à 50% en poids du composant a1) et 25 à 75% en poids du composant a2), à chaque fois par rapport au poids total de la composition.

8. Composition selon la revendication 7, la composition contenant 10 à 50% en poids de méthacrylate d'hydroxyéthyle en tant que composant a1) et 25 à 75% en poids du composant a2), à chaque fois par rapport au poids total de la composition.

9. Composition selon l'une quelconque des revendications 1 à 5, la composition contenant 65 à 85% en poids du système à plusieurs composants a) et 1 à 20% en poids d'un agent colorant en tant que composant d2).

10. Composition selon la revendication 9, la composition contenant 10 à 60% en poids du composant a1), 25 à 75% en poids du composant a2) et 2 à 10% en poids d'un agent colorant en tant que composant d2), à chaque fois par rapport au poids total de la composition.

11. Composition selon la revendication 9 ou 10, la composition contenant 10 à 60% en poids de méthacrylate d'hydroxyéthyle en tant que composant a1), 25 à 75% en poids du composant a2), 0,1 à 2% en poids de butylhydroxytoluène en tant que composant d4) et 2 à 10% en poids d'un agent colorant en tant qu'autre composant d2), à chaque fois par rapport au poids total de la composition.

12. Système pour le traitement de l'ongle d'un doigt ou d'un orteil, comprenant les composants de système :
S1) une composition telle que définie dans l'une quelconque des revendications 6 à 8,
S2) une composition telle que définie dans l'une quelconque des revendications 9 à 11.

13. Procédé pour le traitement cosmétique de l'ongle d'un doigt ou d'un orteil, contenant les étapes de procédé:
I) application d'une couche d'une composition, telle que définie dans l'une quelconque des revendications 6 à 8, sur la surface de l'ongle d'un doigt ou d'un orteil et durcissement consécutif de cette couche par rayonnement électromagnétique avec obtention d'une couche de base durcie;
II) application d'une couche d'une composition, telle que définie dans l'une quelconque des revendications 9 à 11, sur la couche durcie obtenue dans l'étape de procédé I) et durcissement consécutif de cette couche par rayonnement électromagnétique avec obtention d'une couche colorée durcie;
III) application d'une couche d'une composition, telle que définie dans l'une quelconque des revendications 6 à 8, sur l'autre couche durcie obtenue dans l'étape de procédé II) et durcissement consécutif de cette couche par rayonnement électromagnétique avec obtention d'une couche brillante durcie.

14. Procédé selon la revendication 13, l'étape de procédé II) étant réalisée au moins deux fois.

15. Procédé selon la revendication 13 ou 14, le rayonnement électromagnétique étant un rayonnement UV.

16. Procédé selon l'une quelconque des revendications 13 à 15, la couche externe durcie obtenue après l'étape de procédé III) étant traitée par une composition de nettoyage après l'étape de procédé III).
